Europäisches Patentamt

European Patent Office

Office européen des brevets

(11) Publication number: **0 409 294 A1**

# EUROPEAN PATENT APPLICATION

(21) Application number: 90201465.3

(22) Date of filing: 07.06.90

(51) Int. Cl.⁵: **C07G 17/00**, C12N 9/02, C12P 21/00

(30) Priority: 17.07.89 GB 8916289

(43) Date of publication of application:
23.01.91 Bulletin 91/04

(84) Designated Contracting States:
DE GB IT

(71) Applicant: **FARMITALIA CARLO ERBA S.r.L.**
**Via Carlo Imbonati 24**
**I-20159 Milano(IT)**

(72) Inventor: **Kreil, Günther**
**Neu Anif 105B**
**A-5081 Salzburg(AT)**
Inventor: **de Castiglione, Roberto**
**Via Domenichino, 38**
**I-20100(IT)**
Inventor: **Mollay, Christa**
**Schiemerstrasse 8**
**A-5400 Hallein(AT)**
Inventor: **Gozzini, Luigia**
**Viale Stelvio 27/4**
**I-20100 Milano(IT)**
Inventor: **Perego, Rita**
**Via Pasteur 17**
**I-20100 Milano(IT)**
Inventor: **Martinelli, Edoardo**
**Via Mameli, 32**
**Busto Arsizio (Varese)(IT)**

(74) Representative: **Ferrario, Vittorino**
**c/o Farmitalia Carlo Erba S.r.l. Via Carlo**
**Imbonati 24**
**I-20159 Milano(IT)**

(54) Cofactor for the PAM enzyme.

(57) The present invention relates to a cofactor for the enzyme peptidylglycine α-amidating monooxygenase (PAM enzyme), its preparation and purification and its use in the amidation of glycine-extended peptides.

EP 0 409 294 A1

## COFACTOR FOR THE PAM ENZYME

The present invention relates to a cofactor for the enzyme peptidylglycine α-amidating monooxygenase (PAM enzyme), its preparation and purification and its use in the amidation of glycine-extended peptides.

About half of the biologically active peptides isolated from various animal species have been found to terminate with an amide rather than with a free α-carboxyl group. Studies on the cell-free synthesis of prepromelittin, a venom constituent of honeybees, have suggested that a glycine residue is somehow essential for the formation of these terminal amides. The analysis of many precursors of amidated peptides has subsequently shown that invariably a glycine is present after the amino acid which becomes the amidated carboxy terminus in the final product.

It was then demonstrated that the mammalian pituitary gland contains an enzyme, termed peptidylglycine-α-amidating monooxygenase (PAM) which converts peptides with a carboxy-terminal glycine to the corresponding amide. In this reaction, the nitrogen of the glycine is retained in the peptide. This made it likely that these amides are formed by cleavage of an N-C bond via a redox reaction. Further experiments with this pituitary enzyme have shown that the reaction requires oxygen, ascorbate and copper ions. Furthermore, the presence of catalase in the reaction mixture is required.

The amidating enzyme has also been detected in a number of other mammalian tissues, including the parotid and submaxillary gland and heart atrium, as well as in skin of the frog Xenopus laevis . The skin of amphibia often contains large quantities of peptides related or identical to mammalian hormones and/or neurotransmitters. Many of these peptides, which may be present in quantities of several micromoles per gram of skin, are amidated at the carboxy end. For some of these peptides, it has in fact been shown that, in their respective precursors, a glycine residue is present adjacent to the amino acid which forms the COOH-end of the final product.

These peptides are formed in specialized skin glands which have been studied most extensively in the case of Xenopus laevis . The presence of PAM in skin secretion as well as in skin extracts of this frog has been demonstrated previously. Using recombinant DNA techniques, the amino acid sequence of two forms of PAM from Xenopus skin and bovine pituitary has recently been determined.

The PAM enzymes isolated from different sources show similar characteristics. They are apparently copper-containing enzymes with a pH-optimum around 7, requiring ascorbic acid as a redox catalyst. However, the reaction rates obtained with these enzyme preparations have consistently been very low, at most a few nanomoles of product/hour/mg of protein at 37° C.

We have found earlier that the frog PAM enzyme has a lower pH-optimum than PAM from mammalian pituitary glands and that other redox compounds like tetrahydropterin and dihydroxyphenylalanine are about as active as ascorbic acid. The crude skin enzyme was also found to be active with a variety of ions other than copper, while more purified fractions did not require any addition of heavy metal ions for maximal activity.

Furthermore, in the course of PAM purification we observed that a fraction from from skin secretion stimulated the amidation reaction and gave higher yields of amidated peptide than those observed with different combinations of ascorbate, tetrahydropterin, various ions, etc. This fraction was itself devoid of PAM enzyme activity. This fraction contains an additional compound or "cofactor" for the reaction catalyzed by the PAM amidating enzyme.

Accordingly, the present invention provides a substance which acts as a cofactor for the enzyme peptidylglycine α-amidating monooxygenase (PAM) and which has the following properties:

(i) no absorption spectrum above 225 nm;

(ii) is not a peptide;

(iii) is not ninhydrin-positive;

(iv) molecular weight is less than 1000 Da; and

(v) can be electrodialyzed;

(vi) accumulates upon extraction from aqueous solutions with organic solvents at the interface.

The invention also provides a process for the preparation of the PAM cofactor, which process comprises:

(a) obtaining a crude PAM preparation;

(b) digesting the preparation with a proteinase;

(c) filtering the digest over charcoal and collecting the void volume which is eluted;

(d) subjecting the void volume to chromatography.

(e) subjecting the cofactor to electrodialysis;and

(f) isolation of cofactor from the interface that forms between water and e.g. n-hexane or chloroform.

Even though the cofactor has a low molecular weight, it dialyses very slowly in the presence of proteins. In the skin secretion of Xenopus laevis , the cofactor apparently occurs in bound form, i.e. bound to PAM and possibly other proteins. However, some cofactor activity can be recovered in the supernatant after adding to the secretion ammonium sulphate up to 90% saturation.

The crude PAM preparation is obtained by fractionation of skin secretion from X. laevis with ammonium sulphate and subsequent dialysis. The PAM enzyme can then be purified further by chromatography, using for example Sephacryl (Trade Mark) S-200 (Pharmacia).

Having obtained a crude PAM preparation, the cofactor can be liberated by first digesting the proteins and peptides with a proteinase, for example proteinase K or another proteinse with a broad specificity. The proteinase may be used at pH 7.0. The digestion procedure can take place for from 3 to 6 hours.

Next, the enzymatic digest is subjected to chromatography on a charcoal column. The cofactor elutes with the void volume. This fraction is then subjected to further chromatography, again on charcoal or, for example, on S-Sepharose (Trade Mark, Pharmacia) in 2% acetic acid. Alternatively, cofactor can be electrodialyzed or segregated from an aqueous solution by extraction with n-hexane (or chloroform) to the interface between the aqueous and organic layer. Using this procedure, PAM cofactor free of proteins, amino acids, lipids and aromatic organic compounds can be produced.

The PAM cofactor can also be isolated from crude PAM preparations obtained from other sources. Using crude PAM preparations isolaed from bovine heart atrium or queen bee venom glands, the cofactor could be isolated by the same procedure as outlined above.

The PAM cofactor is used according to the invention for the preparation of a COOH-terminally $\alpha$-amidated peptide or protein. The process according to the invention comprises treating a peptide or protein having a COOH-terminal glycine residue, typically at pH about 5.5 (in, for example, 100 mmolar ammonium acetate), with PAM in the presence of the PAM cofactor, oxygen, ascorbate and catalase.

The PAM cofactor can therefore be used in the preparation of a peptide or protein of formula (I):

Y-X-NH$_2$    (I)

wherein X represents the carboxy-terminal amino acid residue of the peptide or protein and Y represent the remainder of the peptide or protein. Such a peptide can be obtained according to the invention from a peptide or protein of formula (II):

Y-X-Gly    (II)

where X and Y are as defined above.

The following Examples illustrate the invention. A Reference Example is provided.

Reference Example: Purification of PAM from Xenopus laevis

The PAM was partially purified from skin secretion of X. laevis . The animals were kept in aerated tanks and forced to release their skin secretion by mild electric shocks of about 15 Volt. The animals were then rinsed with distilled water or physiological saline. This crude skin secretion, which was collected from the animals every three weeks, was the starting material for the preparation of PAM as well as for the PAM cofactor.

The partially purified preparations of PAM were obtained as follows:

1) Skin secretion was collected in 0.9% NaCl and stored frozen.

2) Proteins precipitating between 25 and 65% saturated ammonium sulphate were collected by centrifugation.

3) The precipitate was dissolved in 50 mM ammonium acetate and dialysed against the same buffer overnight.

4) Chromatography on Sephacryl S-200 or S-300 (Pharmacia) in the same buffer yielded a broad peak eluting in the molecular weight range of around 60000 Da.

5) Lyophilization of active fractions.

6) Re-chromatography on Sephacryl S-200 (as in 4).

7) Lyophilization of active fractions.

8) Proteins dissolved in 0.1 molar sodium borate buffer, pH 9 and dialyzed against the same buffer.

9) The dialysed protein was again adjusted to 5.5 with acetic acid and dialysed against 50 mM sodium acetate, pH 5.5.

10) Insoluble material was removed by centrifugation.

Example 1: Preparation of PAM cofactor from Xenopus laevis

1) Skin secretion of X. laevis was fractionated with ammonium sulphate (steps 1-3 in Reference Example).

2) The dialysed solution was adjusted to pH 7, proteinase K was added (30 μg/ml) and incubated for 3-5 hours at room temperature.

3) The digest was then passed twice over an appropriately sized column filled with activated charcoal.

4) The material eluting with the void volume was lyophilized and then re-dissolved in water.

5) For further purification to remove traces of ninhydrin-positive material, the cofactor was passed over S-Sepharose (Pharmacia) in 2% acetic acid.

Example 2: Preparation of PAM cofactor from Xenopus laevis

1) Preparation of crude PAM, steps 1-6 as in Reference Example.

2) Purification of cofactor from this material as in Example 1, steps 2-5.

## Example 3: Preparation of PAM cofactor from Xenopus leavis

1) Preparation of cofactor as in Example 2.

2) Cofactor lyophilized after step 5 in Example 2 was dissolved in 0.65 M ammonium carbonate buffer, pH 8.5 and electrodialyzed at 20 v for 3-5 hours at 4°C. Cofactor migrating to the positive chamber was lyophilized several times.

## Example 4: Preparation of PAM cofactor from Xenopus leavis

1) Preparation of cofactor as in Example 3.

2) Cofactor was dissolved in water and extracted several times with n-hexane (or chloroform).

3) Interface between water and organic layer was recovered, dried and dissolved in water. Cofactor accumulates at the interface indicating its amphiphilic character.

## Example 5: Preparation of PAM cofactor from atrium of bovine heart

It has recently been shown that heart atrium contains PAM activity (Eipper et al , J. Biol. Chem. 263 8371-8379, 1988). A crude granule fraction was prepared from bovine heart atrium (Eipper et al , 1988; de Bold, Can. J. Physiol. Pharmacol. 60 324-330, 1982). This fraction was then used as the starting material for the preparation of the PAM cofactor. The purification was essentially as described for the frog skin secretion and included:

1) Preparation of a crude granule fraction from bovine atrium as described by Eipper et al . (1988).

2) Digestion with proteinase K (as in step 2, Example 1).

3) Chromatography over activated charcoal.

4) Lyophilization.

The resulting preparation had PAM cofactor activity and showed the same characteristics as the preparation from frog skin secretion. These were:

1) absence of ninhydrin-positive material;

2) no spectrum above 225 nm;

## Example 6: Detection of PAM cofactor activity in venom glands of honeybees.

The venom of honeybees, Apis mellifera , contains melittin as the main component, which comprises more than 50% of the dry weight. This peptide of 26 amino acid residues is amidated at the carboxy end. In extracts from venom glands of newly emerged queen bees, we detected PAM activity. The preparation of PAM cofactor from venom glands was as follows:

1) Venom glands from 100 queen bees were disrupted with glass powder.

2) Soluble material was extracted with 50 millimolar ammonium acetate buffer, pH 5.5.

3) Proteins precipitating between 25 and 65% saturated ammonium sulphate were collected by centrifugation.

4) The precipitate was digested with proteinase K and passed over activated charcoal (steps 2-4 of Example 1).

## Example 7 : Influence of the PAM cofactor on the formation of terminal amides

Using partially purified PAM from frog skin in the presence of ascorbate (1 mM) and catalase (100 μg/ml) at pH 5.5, the formation of peptidyl-amide from peptidyl-glycine substrates was very low. For different enzyme preparations, this was found to be of the order of 1 nanomol product/hour/mg protein or less. Addition of PAM cofactor increased the yields roughly proportional to the amount of cofactor present in the reaction mixture. In the presence of sufficient amounts of cofactor, yields in excess of 1 micromol product/min/mg protein were routinely obtained.

## Example 8: Chemical nature and characteristics of the PAM cofactor

Experimental evidence indicated that the PAM cofactor preparations have the following properties:

- no absorption spectrum above 225 nm
- no peptidic material
- no ninhydrin positive material
- low molecular weight (a mass of less than 1000 Da as indicated by the high mobility on high voltage paper electrophoresis)
- upon electrodialysis migrates to the positive chamber,
- can be segregated from an aqueous solution by extraction with n-hexane (or chloroform) to accumulate at the interface suggesting an amphiphilic structure.
- the cofactor preparations did not replace reducing agents like ascorbate, tetrahydropterin or dihydroxyphenylalanine nor catalase in the amidation reaction.
- kinetic analysis has shown that the cofactor enhances the maximal rate of the reaction but has no influence on the Michaelis constant, i.e. on the affinity of the enzyme for the substrate.

- cofactor was used up or inactivated in the course of the reaction and could not be re-activated by addition of, e.g. more ascorbate, etc.

## Claims

1. A substance which acts as a cofactor for peptidylglycine $\alpha$-amidating monooxygenase (PAM) enzyme and which has the following properties:

(i) no absorption spectrum above 225 nm;

(ii) is not a peptide;

(iii) is not ninhydrin-positive;

(iv) molecular weight is less than 1000 Da; and

(v) migrates to positive pole upon electrodialysis;

(vi) accumulates at interface between water and n-hexane or chloroform, a behaviour that indicates an amphiphilic structure.

2. A process for the preparation of a substance as defined in claim 1, which process comprises

(a) obtaining a crude PAM preparation;

(b) digesting the preparation with a proteinase;

(c) filtering the digest over charcoal and collecting the void volume which is eluted; and

(d) subjecting the void volume to chromatography.

(e) separation by electrodialysis;

(f) collect material accumulating at interface between water and organic solvent like n-hexane or chloroform.

3. A process for the preparation of a C-terminal $\alpha$-amidated peptide or protein, which process comprises treating a peptide or protein having a C-terminal glycine residue with PAM in the presence of a substance as defined in claim 1.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 305 063 (KISSEI PHARMACEUTICAL CO., LTD) * Page 3, lines 25-54 * | 1-3 | C 07 G 17/00 C 12 N 9/02 C 12 P 21/00 |
| A | FEBS LETTERS, vol. 202, no. 2, July 1986, pages 251-254; C. MOLLAY et al.: "Detection and partial characterization of an amidating enzyme in skin secretion of Xenopus laevis" * Page 253 * | 1-3 | |
| A | PROC. NATL. ACAD. SCI. USA, vol. 80, August 1980, pages 5144-5148; B.A. EIPPER et al.: "Identification in pituitary tissue of a peptide alpha-amidation activity that acts on glycine-extended peptides and requires molecular oxygen, copper, and ascorbic acid" * The abstract * | 1-3 | |

**TECHNICAL FIELDS SEARCHED (Int. Cl.5)**

C 12 N
C 12 P

The present search report has been drawn up for all claims

| Place of search | Date of completion of the search | Examiner |
|---|---|---|
| THE HAGUE | 22-10-1990 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)